# FASCICULE DE BREVET EUROPEEN

(11) **EP 4 146 308 B1**
(45) Date de publication et mention de la délivrance du brevet: **30.07.2025**
(21) Numéro de dépôt: 21732415.1
(22) Date de dépôt: 03.05.2021
(51) Int. Cl.: A61M 15/00, A61M 15/08, A61M 11/00, A61M 5/145

(54) **DISPOSITIF DE DISTRIBUTION DE PRODUIT FLUIDE**
VORRICHTUNG ZUR ABGABE EINES FLÜSSIGEN PRODUKTS
DEVICE FOR DISPENSING A FLUID PRODUCT

(30) Priorité: 05.05.2020 FR 2004456
(43) Date de publication de la demande: 15.03.2023
(73) Titulaire: APTAR France SAS, 27110 Le Neubourg (FR)
(72) Inventeur: BAILLIE, Mickaël, 27400 Acquigny (FR); MOIA, Florent, 69009 Lyon (FR)
(74) Mandataire: CAPRI
(86) Numéro de dépôt international: PCT/FR2021/050756
(87) Numéro de publication internationale: WO 2021/224571

(56) Documents cités:
- WO-A1-2019/195944
- DE-U1- 20 022 559
- FR-A1- 2 970 955
- US-A1- 2019 126 303

## Description

La présente invention concerne un dispositif de distribution de produit fluide. Plus particulièrement, la présente invention concerne un dispositif unidose ou bidose de distribution nasale de produit fluide pour distribuer un produit fluide pharmaceutique dans le nez d'un utilisateur par l'intermédiaire d'une seule ou de deux pulvérisation(s) nasale(s).

Un inconvénient des dispositifs unidose de distribution nasale de produit fluide existants concerne la variabilité de la dose entre différents dispositifs, notamment en raison des tolérances de fabrications des différentes pièces qui déterminent la position de fin de dose. De plus, la fin de dose est généralement formée par le contact entre le bouchon en élastomère et le fond du réservoir, ce qui implique une pièce déformable, ce qui augmente encore la variabilité de la dose. Le même problème se pose dans un bidose, en particulier concernant la seconde dose.

Le document US2019126303 décrit un dispositif de l'état de la technique.

La présente invention a pour but de fournir un dispositif de distribution de produit fluide qui ne reproduit pas les inconvénients susmentionnés.

Plus particulièrement, la présente invention a pour but de fournir un dispositif de distribution de produit fluide qui réduit la variabilité de dose entre différents dispositifs.

La présente invention a également pour but de fournir un tel dispositif dans lequel la position de fin de dose est définie par seulement des pièces rigides du dispositif.

La présente invention a encore pour but de fournir un tel dispositif dans lequel la position de fin de dose est indépendante de la force d'actionnement exercée par l'utilisateur.

La présente invention a également pour but de fournir un tel dispositif qui soit simple et peu coûteux à fabriquer et à assembler.

La présente invention a donc pour objet un dispositif de distribution de produit fluide comportant un réservoir contenant du produit fluide, une tête de distribution pourvue d'un orifice de distribution, un bouchon-piston déplaçable dans ledit réservoir entre une position de repos et une position de fin d'actionnement pour distribuer du produit fluide à travers ledit orifice de distribution lors d'un actionnement, ledit réservoir étant formé par un corps creux et borgne, comportant un fond et une seule ouverture qui, en position de repos, est obturée de manière étanche par ledit bouchon-piston, ledit dispositif comportant un insert creux inséré et fixé dans ladite tête de distribution, ledit insert supportant une canule pour percer ledit bouchon-piston et ainsi relier ledit réservoir audit orifice de distribution lors de l'actionnement, ledit insert comportant au moins une projection latérale réalisée d'une pièce monobloc et non amovible avec l'insert, pourvue d'une surface inférieure, ladite surface inférieure coopérant en fin d'actionnement avec ledit réservoir pour définir la position de fin d'actionnement dudit piston-bouchon, dans laquelle ledit piston-bouchon ne touche pas le fond dudit réservoir.

Avantageusement, ledit insert comporte une projection latérale périphérique.

En variante, ledit insert comporte plusieurs projections latérales séparées, réparties sur la périphérie dudit insert.

Avantageusement, ladite ouverture dudit réservoir est formée par un col pourvu d'une surface supérieure, ladite surface supérieure coopérant en fin d'actionnement avec ladite surface inférieure de ladite au moins une projection latérale pour définir la position de fin d'actionnement.

Avantageusement, ladite au moins une projection latérale est disposée par rapport audit réservoir de telle sorte que le contact de fin de dose se forme juste avant que ledit piston-bouchon ne touche le fond dudit réservoir.

Avantageusement, ledit réservoir contient une seule dose de produit fluide distribuée en un seul actionnement.

En variante, ledit réservoir contient deux doses de produit fluide distribuées en deux actionnements successifs.

Avantageusement, ladite projection latérale coopère avec ledit réservoir après distribution de la seconde dose.

Ces caractéristiques et avantages et d'autres de la présente invention apparaitront plus clairement au cours de la description détaillée suivante, faite en référence aux dessins joints, donnés à titre d'exemples non limitatifs, et sur lesquels:
La figure 1 est une vue schématique en section transversale d'un dispositif de distribution de produit fluide selon un mode de réalisation avantageux, en position de repos avant actionnement, et
La figure 2 est une vue de détail en section transversale d'une tête de distribution et d'un insert selon un autre mode de réalisation avantageux, en position actionnée.

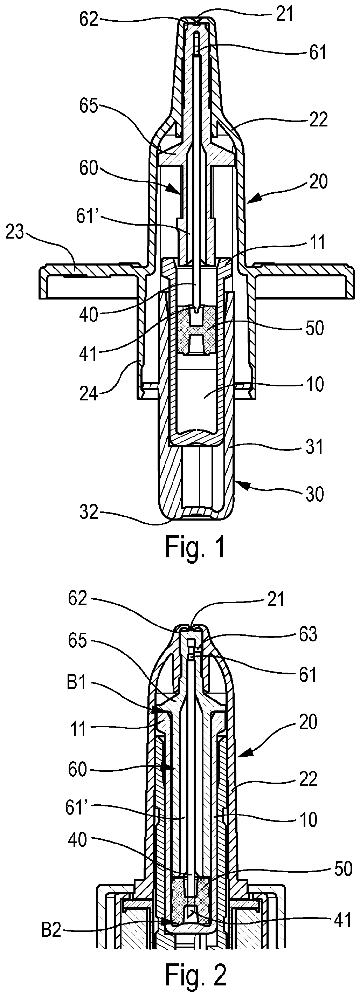

Les termes "proximal" et "distal" se réfèrent à l'orifice de distribution. Les termes "axial" et "radial" se réfèrent à l'axe central longitudinal du dispositif. Les termes "amont" et "aval" se réfèrent à la direction d'écoulement du fluide lors de l'actionnement. Les termes "haut" et "bas" se réfèrent à la position droite du dispositif représentée sur la figure 1.

En référence aux figures, il est représenté un mode de réalisation avantageux de la présente invention, qui est un unidose, c'est-à-dire que le réservoir contient une seule dose de produit fluide distribuée en un seul actionnement. La présente invention s'applique toutefois aussi aux dispositifs de type bidose, dans lesquels le réservoir contient deux doses distribuées en deux actionnements successifs.

Dans ce mode de réalisation, un réservoir 10 contenant du produit fluide à distribuer, typiquement un liquide, est disposé à l'intérieur d'un corps formant une tête de distribution 20. Cette tête de distribution 20 comporte un orifice de distribution 21 orienté axialement. Cet orifice de distribution 21 sert à distribuer une dose de produit fluide hors de ladite tête de distribution 20 lors de l'actionnement du dispositif par un utilisateur.

La tête de distribution 20 comporte un embout nasal allongé 22 comportant à son extrémité axiale proximale ledit orifice de distribution 21, ainsi qu'un corps latéral 24, relié audit embout nasal 22 au niveau d'une bride radiale 23.

Un insert creux 60 est disposé dans la tête de distribution 20, en amont dudit orifice de distribution 21, ledit insert creux 60 définissant un canal d'expulsion 61, 61' et, en coopération avec la paroi de fond de la tête de distribution 20, un profil de pulvérisation 62 directement en amont dudit orifice de distribution 21. Au moins une ouverture radiale 63, disposée à proximité de l'extrémité proximale de l'insert 60, permet au fluide de passer du canal d'expulsion 61 au profil de pulvérisation 62. Le canal d'expulsion comporte une partie de canal proximale 61, au niveau de ladite ouverture radiale 63. Le canal d'expulsion comporte une partie de canal distale 61' de diamètre supérieur à celui de la partie de canal proximale 61.

Selon l'invention, sur sa paroi externe, l'insert 60 comporte une projection latérale 65, s'étendant de préférence sur toute la périphérie. Cette projection latérale 65 est réalisée d'une pièce monobloc avec l'insert 60 et n'est pas détachable ou amovible dudit insert, comme le serait par exemple un pont sécable. La projection latérale 65 comporte une surface supérieure et une surface inférieure.

Une canule creuse 40 est insérée et fixée dans la partie proximale 61 dudit canal d'expulsion, ladite canule 40 comportant à son extrémité distale une pointe de perçage 41. Avantageusement, la canule 40 est emmanchée à force dans la partie de canal proximale 61. Ainsi, l'insertion de la canule 40 dans l'insert 60 est facilitée, l'emmanchement à force étant limitée à la partie de canal proximale 61.

Le réservoir 10 est formé par un corps creux et borgne, comportant une seule ouverture fermée par un bouchon-piston 50. Dans l'exemple représenté, qui est un unidose, le réservoir 10 contient une seule dose de produit fluide à distribuer un actionnement unique du dispositif. Dans le cas d'un bidose, le réservoir contient deux doses, et le dispositif comporte des moyens de fractionnement de dose pour assurer la distribution d'une seule dose à chaque actionnement. L'ouverture du réservoir est formée par un col 11 pourvu d'une surface supérieure. Le piston-bouchon 50 est déplaçable dans le réservoir 10 entre une position de repos et une position de fin d'actionnement pour distribuer ledit produit fluide à travers l'orifice de distribution 21 lors de l'actionnement.

Lors de l'actionnement, le réservoir 10 est déplacé axialement vers le haut par rapport à la tête de distribution 20 et donc par rapport à la canule 40. Le bouchon-piston 50 est ainsi poussé contre la pointe 41 de la canule 40 pour être percé, ce qui permet l'expulsion de tout ou partie du produit fluide contenu dans le réservoir 10.

Des moyens d'actionnement 30 sont prévus pour permettre l'actionnement du dispositif.

Dans le mode de réalisation de la figure 1, ces moyens d'actionnement 30 comportent un corps d'actionnement 31 mobile par rapport à la tête de distribution 20, ledit corps d'actionnement 31 coopérant avec ledit réservoir 10 pour le déplacer axialement par rapport à la tête de distribution 20, en direction de l'orifice de distribution 21.

Le fonctionnement du dispositif unidose représenté sur les figures va être détaillé ci-après.

De manière classique, l'utilisateur place deux doigts sur la bride radiale 23 formée sur la tête de distribution 20, et appuie avec le pouce sur le fond axial distal 32 dudit corps d'actionnement 31. Lors d'un tel actionnement, le réservoir 10 est donc poussé axialement en direction de l'orifice de distribution 21, de sorte que la canule 40 perce le bouchon-piston 50. Le contenu du réservoir 10 est alors relié à l'orifice de distribution 21 et l'appui de l'utilisateur sur le corps d'actionnement 31 déplace le bouchon-piston 50 dans le réservoir 10 pour assurer la distribution du produit fluide.

La position de fin d'actionnement est définie par la butée mécanique entre la surface supérieure du col 11 du réservoir 10 et la surface inférieure de la projection latérale 65 de l'insert 60.

Ainsi, comme visible sur la figure 2, la position de fin d'actionnement est définie par seulement deux pièces rigides du dispositif qui forment une butée B1 bloquant le déplacement axial du réservoir 10 lors de l'actionnement. Cette position de fin d'actionnement est par conséquent indépendante de la force d'actionnement exercée par l'utilisateur pour déplacer le réservoir 10.

La projection latérale 65 est disposée par rapport au réservoir 10 de telle sorte que le contact B1 de fin de dose se forme juste avant que le piston-bouchon 50 ne touche le fond du réservoir 10.

Ainsi, au niveau de B2 sur la figure 2, il n'y a pas de contact et donc pas de compression ou déformation du piston-bouchon 50 en fin de dose, susceptible de générer une variabilité de dose.

Dans les exemples représentés sur les figures, les surfaces de butée du réservoir 10 et de la projection latérale 65 sont sensiblement horizontale dans la position droite représentée sur les figures. En variante, on pourrait imaginer des surfaces inclinées de manière complémentaires. On pourrait aussi imaginer une pluralité de projections latérales 65 non périphériques, par exemple deux, trois ou quatre projections réparties sur la périphérie de l'insert 60, et coopérant en position de fin de dose avec la surface supérieure du col 11 du réservoir 10.

La présente invention fournit donc un dispositif pour lequel la position de fin d'actionnement est définie uniquement par deux pièces rigides qui viennent en butée l'une contre l'autre en fin de course d'actionnement. Ceci rend le dispositif plus robuste et fiable, et garantit des performances reproductibles. La variabilité de dose est ainsi réduite d'au moins 25%, avantageusement d'au moins 50%, de préférence d'environ 60% par rapport aux dispositifs unidose ou bidose standards dans lesquels la fin de dose est définie par le contact entre le bouchon élastomère et le fond du réservoir.

Bien entendu, d'autres variantes de réalisation sont également envisageables, sans sortir du cadre de la présente invention, tel que défini par les revendications annexées.

## Revendications

1. Dispositif de distribution de produit fluide comportant un réservoir (10) contenant du produit fluide, une tête de distribution (20) pourvue d'un orifice de distribution (21), un bouchon-piston (50) déplaçable dans ledit réservoir (10) entre une position de repos et une position de fin d'actionnement pour distribuer du produit fluide à travers ledit orifice de distribution (21) lors d'un actionnement, ledit réservoir (10) étant formé par un corps creux et borgne, comportant un fond et une seule ouverture qui, en position de repos, est obturée de manière étanche par ledit bouchon-piston (50), ledit dispositif comportant un insert (60) creux inséré et fixé dans ladite tête de distribution (20), ledit insert (60) supportant une canule (40) pour percer ledit bouchon-piston (50) et ainsi relier ledit réservoir (10) audit orifice de distribution (21) lors de l'actionnement, **caractérisé en ce que** ledit insert (60) comporte au moins une projection latérale (65), réalisée d'une pièce monobloc et non amovible avec l'insert, pourvue d'une surface inférieure, ladite surface inférieure coopérant en fin d'actionnement avec ledit réservoir (10) pour définir la position de fin d'actionnement dudit piston-bouchon (50), dans laquelle ledit piston-bouchon ne touche pas le fond dudit réservoir.

2. Dispositif selon la revendication 1, dans lequel ledit insert (60) comporte une projection latérale (65) périphérique.

3. Dispositif selon la revendication 1, dans lequel ledit insert (60) comporte plusieurs projections latérales (65) séparées, réparties sur la périphérie dudit insert (60).

4. Dispositif selon l'une quelconque des revendications précédentes, dans lequel ladite ouverture dudit réservoir (10) est formée par un col (11) pourvu d'une surface supérieure, ladite surface supérieure coopérant en fin d'actionnement avec ladite surface inférieure de ladite au moins une projection latérale (65) pour définir la position de fin d'actionnement.

5. Dispositif selon l'une quelconque des revendications précédentes, dans lequel ladite au moins une projection latérale (65) est disposée par rapport audit réservoir (10) de telle sorte que le contact de fin de dose se forme juste avant que ledit piston-bouchon (50) ne touche le fond dudit réservoir (10).

6. Dispositif selon l'une quelconque des revendications précédentes, dans lequel ledit réservoir (10) contient une seule dose de produit fluide distribuée en un seul actionnement.

7. Dispositif selon l'une quelconque des revendications 1 à 5, dans lequel ledit réservoir (10) contient deux doses de produit fluide distribuées en deux actionnements successifs.

8. Dispositif selon la revendication 7, dans lequel ladite projection latérale (65) coopère avec ledit réservoir (10) après distribution de la seconde dose.

## Patentansprüche

1. Vorrichtung zur Abgabe eines fluiden Produkts, aufweisend einen Vorratsbehälter (10), der fluides Produkt enthält, einen Abgabekopf (20), der mit einer Abgabeöffnung (21) versehen ist, einen Kolbenstopfen (50), der in dem Vorratsbehälter (10) zwischen einer Ruhestellung und einer Betätigungsendstellung verschiebbar ist, um bei einer Betätigung fluides Produkt durch die Abgabeöffnung (21) abzugeben, wobei der Vorratsbehälter (10) von einem hohlen, blind endenden Körper gebildet ist, der einen Boden und einer einzigen Öffnung aufweist, die in Ruhestellung durch den Kolbenstopfen (50) dicht verschlossen ist, wobei die Vorrichtung einen hohlen Einsatz (60) aufweist, der in den Abgabekopf (20) eingesetzt und darin befestigt ist, wobei der Einsatz (60) eine Kanüle (40) zum Durchstechen des Kolbenstopfens (50) und damit zum Verbinden des Vorratsbehälters (10) mit der Abgabeöffnung (21) bei der Betätigung trägt, **dadurch gekennzeichnet, dass** der Einsatz (60) mindestens einen seitlichen Vorsprung (65) aufweist, der aus einem einteiligen und mit dem Einsatz nicht lösbaren Teil gebildet ist, versehen mit einer unteren Fläche, wobei die untere Fläche am Ende der Betätigung mit dem Vorratsbehälter (10) zusammenwirkt, um die Betätigungsendstellung des Kolbenstopfens (50) zu definieren, in der der Kolbenstopfen den Boden des Vorratsbehälters nicht berührt.

2. Vorrichtung nach Anspruch 1, wobei der Einsatz (60) einen umlaufenden seitlichen Vorsprung (65) aufweist.

3. Vorrichtung nach Anspruch 1, wobei der Einsatz (60) mehrere getrennte seitliche Vorsprünge (65) aufweist, die auf dem Umfang des Einsatzes (60) verteilt sind.

4. Vorrichtung nach einem der vorstehenden Ansprüche, wobei die Öffnung des Vorratsbehälters (10) von einem Hals (11) gebildet ist, der mit einer oberen Fläche versehen ist, wobei die obere Fläche am Ende der Betätigung mit der unteren Fläche des mindestens einen seitlichen Vorsprung (65) zusammenwirkt, um die Betätigungsendstellung zu definieren.

5. Vorrichtung nach einem der vorstehenden Ansprüche, wobei der mindestens eine seitliche Vorsprung (65) in Bezug auf den Vorratsbehälter (10) derart angeordnet ist, dass der Dosierungsendkontakt unmittelbar bevor Berührung des Bodens des Vorratsbehälters (10) durch den Kolbenstopfen (50) hergestellt wird.

6. Vorrichtung nach einem der vorstehenden Ansprüche, wobei der Vorratsbehälter (10) eine einzige Dosis fluiden Produkts enthält, die mit einer einzigen Betätigung abgegeben wird.

7. Vorrichtung nach einem der Ansprüche 1 bis 5, wobei der Vorratsbehälter (10) zwei Dosen fluiden Produkts enthält, die mit zwei aufeinanderfolgenden Betätigungen abgegeben werden.

8. Vorrichtung nach Anspruch 7, wobei der seitliche Vorsprung (65) nach Abgabe der zweiten Dosis mit dem Vorratsbehälter (10) zusammenwirkt.

## Claims

1. Device for dispensing fluid product comprising a reservoir (10) containing the fluid product, a dispensing head (20) provided with a dispensing hole (21), a stopper (50) which is displaceable in said reservoir (10) between a rest position and an actuation end position to dispense the fluid product through said dispensing hole (21) during an actuation, said reservoir (10) being formed by a hollow, blind body, comprising a base and a single opening which, in the rest position, is closed in a fluid-tight manner by said stopper (50), said device comprising a hollow insert (60) inserted and fixed in said dispensing head (20), said insert (60) supporting a cannula (40) for piercing said stopper (50) and thus connecting said reservoir (10) to said dispensing hole (21) during the actuation, **characterised in that** said insert (60) comprises at least one lateral projection (65), made of a one-piece part and non removable with the insert, provided with a lower surface, said lower surface cooperating at the end of actuation with said reservoir (10) to define the actuation end position of said stopper (50), wherein said stopper (50) does not touche the base of said reservoir.

2. Device according to claim 1, wherein said insert (60) comprises a peripheral lateral projection (65).

3. Device according to claim 1, wherein said insert (60) comprises several separate lateral projections (65), distributed over the periphery of said insert (60).

4. Device according to any one of the preceding claims, wherein said opening of said reservoir (10) is formed by a neck (11) provided with an upper surface, said upper surface cooperating at the end of actuation with said lower surface of said at least one lateral projection (65) to define the actuation end position.

5. Device according to any one of the preceding claims, wherein said at least one lateral projection (65) is disposed with respect to said reservoir (10), such that the dose end contact is formed just before said stopper (50) touches the base of said reservoir (10).

6. Device according to any one of the preceding claims, wherein said reservoir (10) contains a single fluid product dose dispensed in a single actuation.

7. Device according to any one of claims 1 to 5, wherein said reservoir (10) contains two fluid product doses dispensed in two successive actuations.

8. Device according to claim 7, wherein said lateral projection (65) cooperates with said reservoir (10) after dispensing of the second dose.
